# EUROPÄISCHE PATENTSCHRIFT

(11) **EP 0 359 118 B1**
(45) Veröffentlichungstag und Bekanntmachung des Hinweises auf die Patenterteilung: **09.06.1993**
(21) Anmeldenummer: 89116513.6
(22) Anmeldetag: 07.09.1989
(51) Int. Cl.: C07C 43/315, C07C 41/48

(54) **Verfahren zur Herstellung von 1,1,2-Trialkoxyethanen**
Method for the production of 1,1,2-trialkoxyethanes
Procédé pour la fabrication d'éthanes-1,1,2-trialkoxys

(30) Priorität: 15.09.1988 DE 3831327
(43) Veröffentlichungstag der Anmeldung: 21.03.1990
(73) Patentinhaber: BASF Aktiengesellschaft, 67063 Ludwigshafen (DE)
(72) Erfinder: Koeffer, Dieter, Dr., D-6940 Weinheim (DE); Bertleff, Werner, Dr., D-6806 Viernheim (DE)

(56) Entgegenhaltungen:
- EP-A- 0 257 445
- DE-C- 890 945
- US-A- 2 449 470

## Beschreibung

Die vorliegende Erfindung betrifft ein verbessertes verfahren zur Herstellung von 1,1,2-Trialkoxyethanen durch Umsetzung von Formaldehyddialkylacetalen oder von Verbindungen, die unter den Reaktionsbedingungen Formaldehyddialkylacetale bilden können, mit Kohlenmonoxid, Wasserstoff und Alkanolen in Gegenwart eines Katalysators, bestehend aus einer Cobaltcarbonylverbindung und einem Promotor.

Aus der DE-A-3 627 776 ist bekannt, daß Trialkoxyethane aus Formaldehydacetalen, Kohlenmonoxid, Wasserstoff und Alkanolen hergestellt werden können, wenn ein Cobaltcarbonyl-Katalysator mit einem Phosphin-, Arsin-, Stibin- oder Bismutin-Promotor verwendet wird. Nachteile dieses Verfahrens sind zum einen der relativ hohe Preis der verwendeten Promotoren und zum anderen deren Empfindlichkeit gegenüber Luftsauerstoff.

Aus der US-A-2 449 470 ist bekannt, daß 1,1,2-Trialkoxyethane bei der Umsetzung von Formaldehyddialkylacetalen mit Kohlenmonoxid und Wasserstoff in Gegenwart eines Alkanols und eines suspendierten Cobaltoxid-Katalysators erhalten werden. Der erforderliche Reaktionsdruck liegt bei mindestens 600 bar.

Aus der DE-A-890 945 ist die Darstellung von 1,1,2-Trimethoxyethan und Ethylenglykolmonomethylether aus Formaldehyddimethylacetal und einem Kohlenmonoxid-Wasserstoff-Gasgemisch unter erhöhtem Druck bekannt. Als Katalysator wird Cobaltoxid auf Kieselgel angegeben. Die Selektivität für Trimethoxyethan läßt zu wünschen übrig.

Der Erfindung lag nun die Aufgabe zugrunde, ein verfahren zur Herstellung von 1,1,2-Trialkoxyethanen zu finden, das den vorgenannten Nachteilen abhilft.

Demgemäß wurde ein verbessertes Verfahren zur Herstellung von 1,1,2-Trialkoxyethanen der allgemeinen Formel I 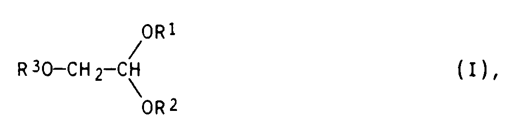
in der R¹ bis R³ unabhängig voneinander C₁- bis C₂₀-Alkyl oder Cycloalkyl bedeuten, oder die Reste R¹ und R² miteinander unter Ausbildung eines 5- bis 7-gliedrigen Ringes verknüpft sein können, durch Umsetzung von Formaldehyddialkylacetalen der allgemeinen Formel II 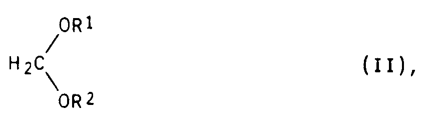
in der R¹ und R² die oben genannten Bedeutungen haben, oder von Verbindungen, die unter den Reaktionsbedingungen Formaldehyddialkylacetale bilden können, mit Kohlenmonoxid, Wasserstoff und Alkanolen R³OH bei erhöhtem Druck und erhöhter Temperatur in Gegenwart eines Katalysators, bestehend aus einer Cobaltcarbonyl-Verbindung und einem Promotor gefunden, das dadurch gekennzeichnet ist, daß der Promotor ein Alkali-, Erdalkali- oder Ammoniumsalz einer protonenaktiven Verbindung mit einer Säurekonstante Kₛ von 10⁻² bis 10⁻¹⁴ oder ein Alkali- oder Erdalkalihydroxid oder eine Aminocarbonsäure ist.

Als Ausgangsverbindungen für das erfindungsgemäße Verfahren dienen Formaldehyddialkylacetale der Formel II 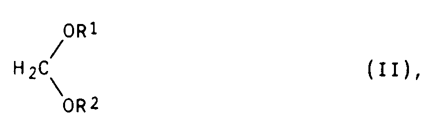
in der die Reste R¹ und R² gleiche oder verschiedene C₁- bis C₂₀-Alkyl-oder Cycloalkyl-Reste bedeuten oder miteinander unter Ausbildung eines 5-bis 7-gliedrigen Ringes verknüpft sind. Vorzugsweise stehen R¹ und R² für primäre oder sekundäre C₁- bis C₈-Alkyl-Reste, insbesondere für primäre C₁- bis C₄-Alkyl-Reste. Als Cycloalkylreste eignen sich C₄- bis C₁₂-Cycloalkyl, vorzugsweise C₄- bis C₈-Cycloalkyl, insbesondere C₅- bis C₈-Cycloakyl.

Als Reste R¹ und R² seien beispielsweise Methyl, Ethyl, Propyl, Isopropyl, Butyl, Isobutyl, Pentyl, Hexyl, Heptyl, Octyl, 2-Ethylhexyl, Cyclopentyl, Cyclohexyl und Cyclooctyl genannt. Cyclische Acetale sind z.B. 1,3-Dioxolan oder 1,3-Dioxan. Anstelle der Acetale II können auch deren Vorläufer, nämlich Formaldehyd oder Verbindungen, die unter den Reaktionsbedingungen Formaldehyd freisetzen, wie Paraformaldehyd oder Trioxan, und die entsprechenden Alkohole eingesetzt werden, wobei das Verhältnis von Aldehyd zu Alkohol in weiten Grenzen schwanken kann. Zweckmäßigerweise wird man pro Mol Aldehyd 1 bis 5 mol Alkohol verwenden. Besonders bevorzugt werden Acetale umgesetzt, deren Alkoholkomponente dem für die Umsetzung verwendeten Alkohol R³OH entsprechen.

Als Alkohole R³OH dienen C₁- bis C₂₀-Alkohole wie Alkanole und Cycloalkanole, vorzugsweise C₁ bis C₈-Alkohole, insbesondere C₁- bis C₄-Alkohole. Beispielsweise seien folgende Alkohole aufgeführt: Methanol, Ethanol, Propanol, Isopropanol, n-Butanol, iso-Butanol, n-Pentanol, Isoamylalkohol, Neopentylalkohol, n-Hexanol, Hexanol-2, Cyclohexanol, n-Heptanol und n-Octanol.

Das Formaldehyddialkylacetal II wird mit mindestens äquimolaren Mengen Alkohol R³OH umgesetzt. Vorteilhaft kann man den Alkohol im Überschuß einsetzen, z.B. 1 bis 5, vorzugsweise 1,1 bis 4, insbesondere 1,5 bis 2,5 mol Alkohol R³OH pro Mol Acetal. Höhere Überschüsse sind möglich, bringen aber keine weiteren Vorteile.

Für die Hydroformylierung der Acetale II wird ein Kohlenmonoxid-Wasserstoffgemisch verwendet, in dem pro Mol Kohlenmonoxid 0,5 bis 1,5 mol, insbesondere 0,5 bis 1 mol Wasserstoff vorliegen. Vorzugsweise liegt das Molverhältnis CO:H₂ bei 1:1.

Das erfindungsgemäße Verfahren wird in Gegenwart eines Katalysators durchgeführt, der aus einer Cobaltcarbonyl-Verbindung und einem Promotor besteht, wobei der Promotor ein Alkali-, Erdalkali- oder Ammoniumsalz einer protonenaktiven Verbindung mit einer Säurekonstante Kₛ von 10⁻² bis 10⁻¹⁴ oder ein Alkali- oder Erdalkalihydroxid oder eine Aminocarbonsäure ist.

Als Cobaltcarbonyl-Verbindung können z.B. Dicobaltoctacarbonyl oder Tetracarbonylhydridocobalt dienen. Die Cobaltcarbonyl-Verbindungen können auch in situ aus Cobalt-Verbindungen, die unter den Reaktionsbedingungen Cobaltcarbonylkomplexe bilden können, wie Cobaltsalze organischer oder anorganischer Säuren, z.B. Cobaltacetat, Cobaltlaurat, Cobalt-2-ethylhexanoat, Cobaltcarbonat, Cobaltnitrat oder Cobalthalogenide, oder aus Cobaltoxid hergestellt werden.

Als Promotoren können verwendet werden Alkali-, Erdalkali- oder Ammoniumsalze, wie z.B. Na₂HPO₄, Na₃PO₄, K₂HPO₄, K₃PO₄, KNH₄HPO₄, Lithiumacetat, Natriumacetat, Kaliumacetat, Magnesiumacetat, Natriumpropionat, Natriumbutyrat, Natrium-2-ethylhexanoat, Ammoniumacetat oder Natriumphenolat oder Alkali- oder Erdalkalihydroxide, wie z.B. Lithiumhydroxid, Natriumhydroxid, Kaliumhydroxid oder Calciumhydroxid, oder Aminocarbonsäuren wie Aminoessigsäure, 2-Aminopropionsäure, 3-Aminopropionsäure oder 2-Aminobuttersäure. Vorzugsweise werden als Promotoren solche Verbindungen verwendet, die im Reaktionsgemisch löslich sind, so z.B. die Salze von Carbonsäuren, wie Lithiumacetat, Natriumacetat, Kaliumacetat, Magnesiumacetat, Kalium-2-ethylhexanoat, Natriumstearat oder Kaliumstearat. Insbesondere werden als Promotoren solche Verbindungen verwendet, die neben den obengenannten Eigenschaften preiswert zur Verfügung stehen müssen, wie z.B. Natriumacetat, Natriumpropionat, Natrium-2-ethylhexanoat oder Aminoessigsäure.

Der Katalysator wird vorteilhaft in situ aus der Cobaltcarbonylverbindung bzw. dessen Vorläufern und dem Promotor hergestellt. Das Molverhältnis Promotor zu Cobalt liegt im allgemeinen bei 0,1 bis 2, vorzugsweise bei 0,3 bis 1,5.

Die Cobaltkonzentration kann zwischen 0,01 und 6 Gew.%, bezogen auf die Reaktionsmischung, gewählt werden. Niedrigere oder höhere Konzentrationen sind auch möglich.

Das erfindungsgemäße Verfahren kann in Gegenwart oder in Abwesenheit von Lösungsmitteln erfolgen. Als Lösungsmittel kommen z.B. Ether wie Diethyl-oder Diphenylether, aromatische oder aliphatische Kohlenwasserstoffe wie Benzol, Toluol oder Hexan und Alkohole in Betracht. Vorteilhaft dienen die für die Umsetzung benötigten Alkohole R³OH zugleich als Lösungsmittel.

Die Umsetzung kann diskontinuierlich oder vorzugsweise kontinuierlich bei einem Druck von 100 bis 700, insbesondere 200 bis 400 bar und einer Temperatur von 50 bis 300, vorzugsweise 100 bis 250°C nach den dafür üblichen Techniken durchgeführt werden.

Anschließend können die Trialkoxyethane I in an sich bekannter Weise, z.B. durch Destillation aus dem Gemisch, isoliert werden.

1,1,2-Trialkoxyethan stellt ein vielseitig verwendbares Zwischenprodukt für organische Synthesen dar. Beispielsweise kann daraus durch Acetalspaltung Alkoxyacetaldehyd hergestellt werden, das durch Kondensation mit Formaldehyd zu Polyolen umgesetzt werden kann. Durch Eliminierung von Alkohol kann weiterhin Dialkoxyethen, z.B. Dimethoxyethen hergestellt werden, das als Ausgangsstoff für Polymerisate dient.

### Beispiele

### Beispiele 1 bis 7

In einem Autoklaven wurden 0,85 mol Methylal und 1,7 mol Methanol in Gegenwart des Katalysators, bestehend aus Dicobaltoctacarbonyl und Promotor, bei 150°C und 280 bar innerhalb von 90 Minuten hydroformyliert, wobei das Verhältnis Kohlenmonoxid zu Wasserstoff 1:1 betrug. Nach Abkühlen auf Raumtemperatur und Entspannen des Autoklaven wurde der Reaktionsaustrag gewogen und gaschromatographisch analysiert. Die Versuche sind in Tabelle 1 zusammengestellt.

**Tabelle 1**

| Hydroformylierung von Methylal | | | | | |
|---|---|---|---|---|---|
| Beispiele | Katalysatorsystem | | Säurekonstante Kₛ der korrespondierenden Säure^{c}) | MethylalUmsatz (%) | TMOE-Selektivität^{a}) (%) |
| | Co₂(CO)₈ (mmol) | Promotor (mmol) | | | |
| 1*) | 3 | - | - | 70 | 35 |
| 2 | 3 | 1,2 NaH₂PO₄ | H₃PO₄ 10^{-2,16} | 68 | 45 |
| 3 | 3 | 1,2 Na₂HPO₄ | H₂PO₄- 10^{-7,21} | 60 | 50 |
| 4 | 3 | 3 NaCH₃COO | CH₃COOH 10^{-4,75} | 61 | 51 |
| 5 | 3 | 6 NaCH₃COO | CH₃COOH 10^{-4,75} | 50 | 59 |
| 6*) | 3 | 15 NaCH₃COO | CH₃COOH 10^{-4,75} | - | - |
| 7^{b}) | 1,5 | 1,5 NH₂CH₂COOH | | 63 | 54 |

| | | | | | |
|---|---|---|---|---|---|
| a) TMOE: 1,1,2-Trimethoxyethan | | | | | |
| b) 250 bar, CO/H₂ 1:1, 150°C, 1,5 h; 0,42 mol Methylal, 0,84 mol Methanol | | | | | |
| c) Lit.: Holleman-Wiberg, Lehrbuch der Anorganischen Chemie, 91.-100. Auflage, Seite 196-199, 241, Verlag Walter de Gruyter, Berlin, New York, 1985. | | | | | |
| *) Vergleichsbeispiele | | | | | |

### Beispiele 8 bis 16

Analog den Beispielen 1 bis 7 wurden 0,4 mol Butylal und 0,8 mol Butanol in Gegenwart von 4 mmol Dicobaltoctacarbonyl und unterschiedlichen Promotoren zur Reaktion gebracht. Die Versuche sind in Tabelle 2 zusammengestellt.

**Tabelle 2**

| Hydroformylierung von Butylal | | | | | | |
|---|---|---|---|---|---|---|
| Bei-spiele | Promotor (mol/mol Co) | Säurekonstante Kₛ der korrespondierenden Säure^{d}) | Butylal-Umsatz (%) | Selektivität (%) | | |
| | | | | TBOE | BEG | BOA |
| 8*) | - | - | 97 | 24 | 19 | 3 |
| 9 | 0,6 KCH₃COO | CH₃COOH 10^{-4,75} | 53 | 58 | 9 | 10 |
| 10 | 0,3 NaOH | H₂O 10⁻¹⁴ | 83 | 52 | 13 | 7 |
| 11 | 0,6 NaOH | H₂O 10⁻¹⁴ | 70 | 50 | 16 | 9 |
| 12 | 0,3 Na₃PO₄ | HPO₄²^{⁻} 10^{-12,3} | 76 | 43 | 15 | 8 |
| 13 | 0,3 Na₂HPO₄ | H₂PO₄- 10^{-7,21} | 91 | 33 | 20 | 6 |
| 14 | 0,6 NaH₂PO₄ | H₃PO₄ 10^{-2,16} | 94 | 30 | 21 | 6 |
| 15*) | 0,6 NaCl | HCl 10^{+7,0} | 98 | 17 | 16 | 2 |
| 16*) | 0,6 Na₂SO₄ | HSO₄- 10^{-1,96} | 94 | 26 | 11 | 5 |
| Abkürzungen: BOA: Butoxyacetaldehyd, TBOE: 1,1,2-Tributoxyethan, BEG: Ethylenglykolmonobutylether | | | | | | |

| | | | | | | |
|---|---|---|---|---|---|---|
| *) Vergleichsbeispiele | | | | | | |
| d) vgl. Fußnote c) in Tabelle 1. | | | | | | |

### Beispiel 17

In einem kontinuierlich von unten nach oben durchströmten Rohrreaktor mit einem Volumen von 360 ml wurden 9800 g einer Reaktionslösung, enthaltend 4870 g (64,1 mol) Methylal, 4360 g (136,3 mol) Methanol, 457,5 g (1,33 mol) Cobalt-2-ethylhexanoat, 65,1 g (0,79 mol) Natriumacetat bei einer mittleren Reaktionszeit von 30 Minuten bei 250 bar und 150°C hydroformyliert. Aus den Einsatzmaterialien enthielt die Lösung eingangs 0,5 % Wasser. Das Verhältnis Kohlenmonoxid zu Wasserstoff betrug 1:1. Der entspannte und abgekühlte Reaktionsaustrag, 10454 g, wies nach gaschromatographischer Analyse folgende Zusammensetzung auf: 3753 g (49,4 mol) Methylal, 3910 g (122,2 mol) Methanol, 1516 g (12,6 mol) 1,1,2-Trimethoxyethan, 314 g (17,4 mol) Wasser, 31,9 g (0,4 mol) Ethylenglykolmonomethylether und 63 g (0,9 mol) Methoxyacetaldehyd sowie nicht näher bestimmte Anteile an höher siedenden Komponenten und den Katalysator.

## Patentansprüche

1. Verfahren zur Herstellung von 1,1,2-Trialkoxyethanen der allgemeinen Formel I 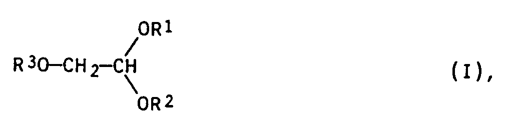 in der R¹ bis R³ unabhängig voneinander C₁- bis C₂₀-Alkyl oder Cycloalkyl bedeuten, oder die Reste R¹ und R² miteinander unter Ausbildung eines 5- bis 7-gliedrigen Ringes verknüpft sein können, durch Umsetzung von Formaldehyddialkylacetalen der allgemeinen Formel II 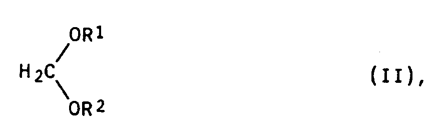 in der R¹ und R² die oben genannten Bedeutungen haben, oder von Verbindungen, die unter den Reaktionsbedingungen Formaldehyddialkylacetale bilden können, mit Kohlenmonoxid, Wasserstoff und Alkanolen R³OH bei erhöhtem Druck und erhöhter Temperatur in Gegenwart eines Katalysators, bestehend aus einer Cobaltcarbonyl-Verbindung und einem Promotor, dadurch gekennzeichnet, daß der Promotor ein Alkali-, Erdalkali- oder Ammoniumsalz einer protonenaktiven Verbindung mit einer Säurekonstante Kₛ von 10⁻² bis 10⁻¹⁴ oder ein Alkali- oder Erdalkalihydroxid oder eine Aminocarbonsäure ist, und man pro mol Cobalt 0,1 bis 2 mal Promotor verwendet.

2. Verfahren nach Anspruch 1, dadurch gekennzeichnet, daß der Promotor ein Alkali-, Erdalkali- oder Ammoniumsalz einer Carbonsäure ist.

3. Verfahren nach Anspruch 1, dadurch gekennzeichnet, daß man Formaldehyd oder Verbindungen, die unter den Reaktionsbedingungen Formaldehyd freisetzen, und die entsprechenden Alkohole verwendet.

## Claims

1. A process for preparing a 1,1,2-trialkoxyethane of the general formula I 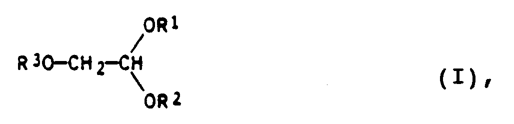 where R¹ to R³ are each independently of the others C₁-C₂₀-alkyl or cycloalkyl, or R¹ and R² may be linked together to form a 5-, 6- or 7-membered ring, by reacting a formaldehyde dialkyl acetal of the general formula II 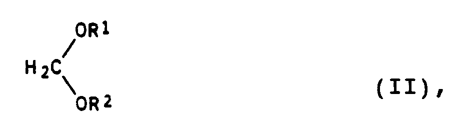 where R¹ and R² are each as defined above, or a compound which is capable of forming a formaldehyde dialkyl acetal under the reaction conditions, with carbon monoxide, hydrogen and an alkanol R³OH under superatmospheric pressure at elevated temperature in the presence of a catalyst composed of a cobalt carbonyl compound and a promotor, wherein the promotor is an alkali metal, alkaline earth metal or ammonium salt of a protogenic compound having an acid constant Kₐ of from 10⁻² to 10⁻¹⁴, an alkali metal or alkaline earth metal hydroxide or an aminocarboxylic acid, and from 0.1 to 2 moles of promotor are used per mole of cobalt.

2. A process as claimed in claim 1, wherein the promotor is an alkali metal, alkaline earth metal or ammonium salt of a carboxylic acid.

3. A process as claimed in claim 1, wherein formaldehyde, or a compound which liberates formaldehyde under the reaction conditions, and the corresponding alcohol is used.

## Revendications

1. Procédé de préparation de 1,1,2-trialcoxyéthanes de la formule générale I 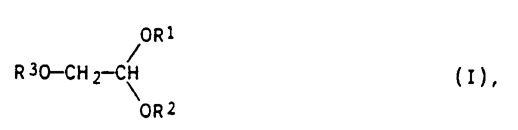 dans laquelle R¹ à R³ représentent, indépendamment les uns des autres, des radicaux cycloalkyle ou alkyle en C₁ à C₂₀, ou bien les symboles R¹ et R² peuvent être mutuellement liés avec formation d'un noyau pentagonal à heptagonal, par la réaction de formaldéhydedialkylacétals de la formule générale II 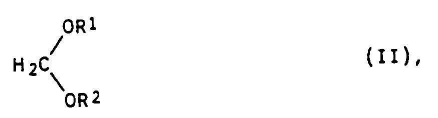 dans laquelle R¹ et R² possèdent les significations qui leur ont été attribuées ci-dessus, ou de composés qui peuvent former des formaldéhydedialkylacétals dans les conditions réactionnelles, avec le monoxyde de carbone, l'hydrogène et des alcanols R³OH, sous pression élevée et température élevée, en présence d'un catalyseur, constitué d'un composé du cobalt-carbonyle et d'un promoteur, caractérisé en ce que le promoteur est un sel de métal alcalin, de métal alcalino-terreux ou d'ammonium d'un composé à protons actifs avec une constante d'acidité Kₛ de 10⁻² à 10⁻¹⁴, ou un hydroxyde de métal alcalin ou de métal alcalino-terreux et on utilise 0,1 à 2 moles de promoteur par mole de cobalt.

2. Procédé suivant la revendication 1, caractérisé en ce que le promoteur est un sel de métal alcalin, de métal alcalino-terreux ou d'ammonium d'un acide carboxylique.

3. Procédé suivant la revendication 1, caractérisé en ce que l'on utilise le formaldéhyde ou des composés qui libèrent du formaldéhyde dans les conditions réactionnelles et les alcools correspondants.
